# EUROPEAN PATENT APPLICATION

(11) **EP 4 830 659 A1**
(43) Date of publication of application: **28.10.2026**
(21) Application number: 25172096.7
(22) Date of filing: 23.04.2025
(51) Int. Cl.: A61B 34/30, A61B 34/35, A61B 34/37, A61B 34/00, A61B 90/00, A61B 90/50, A61B 34/20

(54) **ROBOT ARM FOR USE IN SURGERY, MICROSURGERY OR SUPER-MICROSURGERY**

(71) Applicant: Microsure B.V., 5692 EA Son (NL)
(72) Inventor: VAN DER WALLE, Dirk, 6861 BB Ooosterbeek (NL); OVERWIJK, Charlotte R., 5643 JK Eindhoven (NL)
(74) Representative: WENDE IP GbR

(57) **Abstract**

The present invention relates to a master robot arm (200) for a master control unit (400) of a surgical, microsurgical or super-microsurgical robot system (100).

## Description

The present invention belongs to the technical field of robot systems for use in surgical, microsurgical or super-microsurgical procedures.

In particular, the present invention refers to a master robot arm for a master control unit of a surgical, microsurgical or super-microsurgical robot system.

The present invention further refers to a master control unit and a surgical, microsurgical or super-microsurgical robot system comprising the same.

Microsurgery and super-microsurgery currently play a significant role in medical practice.

In surgical procedures, in particular when microsurgery or super-microsurgery are concerned, robots are used for precise surgical handlings on the submillimeter scale.

The use of robots facilitates accurate movements with micrometer precision without substantial tremor, thereby superseding the limitations of human hand manipulation.

Input to a robot system shall be given with a high level of accuracy, giving the surgeon the feeling of manipulating an instrument with his/her hand.

In particular, robot systems shall provide a sufficient level of dexterity, enabling the user to perform complex movements in the microsurgical or super-microsurgical workspace, at the same time avoiding the risk of collision with other objects (or even the patient) during manipulation.

In general, robot systems for use in surgery, microsurgery or super-microsurgery comprise three main sub-systems, namely a master control, a robotic cart, and a slave robot arm.

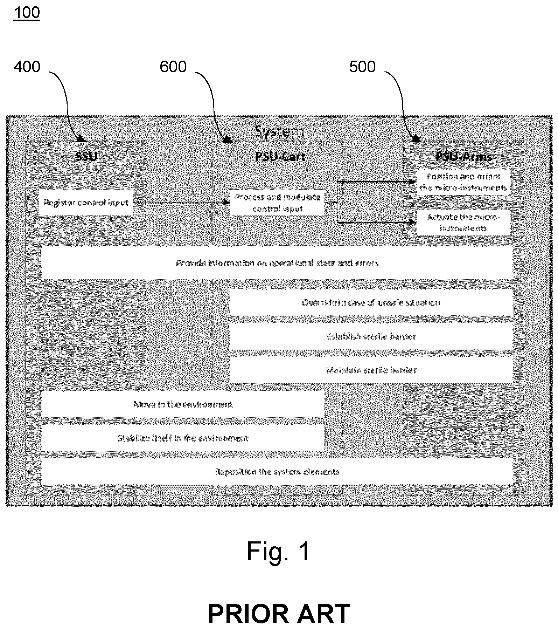

**Fig. 1** schematically illustrates the main functions of said main sub-systems, and how they interact with each other.

The master control unit has two main components, namely a master robot arm and a cart.

The cart allows transporting the master control unit from one location to another, or repositioning the master control unit to a desired position, e.g. within the operatory room.

The master robot arm actuates the slave robot arm to operate and manipulate a surgical instrument, provided at the distal end of the slave robot arm, based on a received input from a user, e.g. a surgeon.

As a non-limiting example, the slave robot arm can be adapted to perform anastomoses.

As a non-limiting example, the surgical instrument can be a hinged surgical instrument, such as a scissors-type or forceps-type surgical instrument.

The robot cart mainly serves for the purpose of moving and/or positioning of the robotic system in the environment, and stabilizing the robotic system in a desired position for surgery.

When a surgical, microsurgical or super-microsurgical operation is carried out, it is of the utmost importance that the master robot arm can be operated by the user with the required level of dexterity, precision, and freedom of movement.

Also, it is important that kinematics of the master robot arm allow actuating the slave robot arm with a high level of precision and reliability.

WO2019220408A1 discloses a master controller assembly for a robotic surgery system, the robotic surgery system further comprising at least one slave robot assembly including a slave surgical instrument provided with at least a slave surgical grip device. The master controller assembly is configured to detect a manual command provided by the user through a master input tool. The master controller assembly further comprises at least one sensing assembly, configured to detect a radially directed pressure action exerted by the user at any point of an operative surface of the master input tool.

In this known solution, the master input tool can be a hand-held manipulandum master tool having a shape which is familiar for most surgeons, e.g. replicating appearance of traditional surgery tweezers.

This gives the surgeon a feeling of manipulating the surgical instrument directly with his/her hands.

Nonetheless, although effective, this known solution still has some drawbacks.

In particular, precision and accuracy in operating the slave robot assembly (and, in turn, the slave surgical instrument) are still sub-optimal.

Hence, there is room for further improvement.

In view of the above, it is an object of the present invention to provide a master robot arm for a master control unit of a surgical, microsurgical or super-microsurgical robot system allowing the surgeon to actuate a slave robot arm with increased level of precision, dexterity, stability, ease of use, and freedom of movement.

The above object is accomplished by the provision of a master robot arm as defined in claim 1.

According to the invention, a master robot arm for a master control unit of a surgical, microsurgical or super-microsurgical robot system, at least comprises:
a first, distal arm element;
a second, intermediate arm element;
a first distal joint having a first distal joint element configured to perform a first roll movement, and a second distal joint element configured to perform a first pitch movement, and
a second, intermediate joint having a first intermediate joint element configured to perform a second pitch movement, and a second intermediate joint element configured to perform a second roll movement.

The present invention provides a master robot arm.

The robot arm is adapted for use in a master control unit, in particular a master control unit of a surgical, microsurgical or super-microsurgical robot system.

In the context of the present invention, the term "proximal" denotes a side which is closer to the user, e.g. a surgeon.

Similarly, the term "distal" denotes a side that is farther from the user.

The master robot arm comprises a first, distal arm element.

Also, the master robot arm comprises a second, intermediate arm element.

Still further, the master robot arm comprises a first distal joint.

The distal joint comprises first distal joint element, configured to perform a first roll movement.

The distal joint element also comprises a second distal joint element, configured to perform a first pitch movement.

There is also a second, intermediate joint provided in the master robot arm.

The intermediate joint comprises first intermediate joint element, configured to perform a second pitch movement.

The intermediate joint further comprises a second intermediate joint element, configured to perform a second roll movement.

This arrangement allows minimizing mechatronic complexity of the master robot arm.

In particular, the invention provides a passive design where no active components, e.g. motors, brakes or the like, are comprised in the master control arm.

The invention is based on the basic idea that, by the provision of a simplified configuration which does not include any active components such as motors or brakes, a master robot arm having an improved kinematics can be obtained, resulting in enhanced dexterity, stability, ease of use, and freedom of movement during operation. Also, the absence of complex, motorized components allow reducing noise and/or heat, and prevent potential points of failure.

Hence, the user's experience in the surgical workspace is significantly improved.

Also, the slave robot arm and, in turn, at least one surgical instrument provided at its distal end, can be operated and manipulated with the required level of accuracy and reliability.

Advantageously, the master robot arm may further comprise a third, proximal arm element.

The proximal arm element provides a user interface allowing the user to provide a user input for operating the master robot arm.

The master robot arm may further include a third, proximal joint.

The proximal joint comprises a first proximal joint element, configured to perform a third pitch movement.

The proximal joint further comprises a second proximal joint element, configured to perform a third roll movement.

The distal joint, the intermediate joint, and the proximal joint provide the master robot arm with six degrees of freedom (DOF).

Accordingly, components of the master robot arm can be orientated in different lateral and rotational directions to provide a 6-DOF-input at the distal end of the slave robot arm, to operate and manipulate at least one surgical instrument connected thereto. Advantageously, the proximal arm element can be in the shape of a joystick.

The joystick provides a hand interface allowing the surgeon to actuate the master robot arm.

The joystick may comprise a pair of hinged levers, configured to perform an opening/closing movement with respect to one another.

This allows providing an additional degree of freedom (DOF).

The opening/closing movement of the levers enables the user to control an opening/closing/pinching operation of the surgical instrument, e.g. a hinged surgical instrument such as a scissors-type or a forceps-type surgical instrument, provided at the distal end of the slave robot arm.

During pinching, the hinged levers are brought from an open, resting state to a closed state.

Then, when pinching is released, the hinged levers automatically return to the former open, resting state.

By using a hinge connecting the levers, a certain degree of haptic feedback for the user can be provided.

Also, the hinge allows mimicking the behavior of a corresponding hinged surgical instrument that is manipulated. Hence, operation of the surgical instrument can be easily and intuitively controlled.

Preferably, the maximum force that the user is enabled to feel when the joystick is in a fully-closed state is comprised between 1.5 N and 2.5 N.

To further enhance the user's convenience, the joystick (and, in particular, the two levers) may be designed to have improved ergonomics and stability.

In particular, the joystick may comprise a 3-point support for placement of the thumb, the index finger, and the thenar web space of the user's hand.

Also, the levers may be provided with slits to intuitively indicate where the user shall place his/her fingers to properly hold and handle the joystick.

Preferably, the joystick is designed so that its maneuverability resembles that of the surgical instrument to be manipulated, especially regarding pinching and diameter. At least one of the joint elements of the master robot arm is provided with an encoder. The encoder can be a magnetic encoder.

Alternatively, the encoder can be an optical encoder.

The encoder allows to reliably sense a position of the joint element.

For instance, the first distal joint element, and/or the second distal joint element, and/or the first intermediate joint element may comprise an optical encoder.

Additionally or alternatively, the second intermediate joint element, and/or the first proximal joint element, and/or the second proximal joint element may comprise a magnetic encoder.

The distal joint may be characterized by the following ranges of motion:
first roll movement: +-inf, and/or
first pitch movement: -5° to +30° with respect to the first pitch rotation axis,

Additionally or alternatively, the intermediate joint may be characterized by the following ranges of motion:
second pitch movement: +62° to +118° with respect to the second pitch rotation axis, and/or
second roll movement: -80° to +80° with respect to the second roll rotation axis,

Additionally or alternatively, the proximal joint may be characterized by the following ranges of motion:
third pitch movement: -80° to +80° with respect to the third pitch rotation axis, and/or
third roll movement: +-inf.

Furter potential values/ranges can be:

| Left·arm·rangen¤ | Right·arm·rangen¤ |
|---|---|
| [0][º]≤K-roll≤[47][º]¤ | [-47][º]≤K-roll≤[0][º]¤ |
| [0][º]≤K-pitch≤[45.5][º]¤ | [0][º]≤K-pitch≤[45.5][º]¤ |
| [90][º]≤L-pitch≤[131][º]¤ | [90][º]≤L-pitch≤[131][º]¤ |
| [-125.4][º]<L-roll:5[35.4][º]¤ | [-35.4][º]≤L-roll≤[125.4][º]¤ |
| [-80][º]≤M-pitch≤[74][º]¤ | [-80][º]≤M-pitch≤[74][º]¤ |
| M-roll=[∞][º]¤ | M-roll=[∞][º]¤ |

The above-specified, preferred ranges of motion for the joints allow optimizing kinematics of the master robot arm.

In order to make the master control unit stable against gravitational component, the master robot arm may comprise at least one gravity compensation means.

For instance, the gravity compensation means can be a spring-loaded gravity compensator.

Preferably, at least one gravity compensation means comprises a first gravity compensation means and a second gravity compensation means.

Preferably, the first gravity compensation means is arranged in the second distal joint element.

Preferably, the second gravity compensation means is arranged in the first intermediate joint element.

This allows successfully compensating for the induced momentum on each joint, generated by the mass of the linkage assembly of the master robot arm.

The gravity compensation means ensures that the arm elements are maintained in place without the need of a constant external force, allowing for smooth and effortless adjustments.

Also, strain on the user and the risk of unintentional movements of the robot arm are minimized, which is important to obtain the required level of precision.

This also enhances the overall safety conditions.

The second intermediate joint element and the second proximal joint element are balanced in a predefined pose.

In particular, this is achieved by tuning the center of mass properties in such a way that these joint elements have a stable pose in a predefined, desired orientation.

Advantageously, the distal arm element may have a length between 250 mm and 400 mm.

Preferably, the distal arm element has a length of 350 mm.

Advantageously, the intermediate arm element may have a length between 250 mm and 400 mm .

Preferably, the intermediate arm element has a length of 350 mm.

The present invention further provides a master control unit for a surgical, microsurgical or super microsurgical robot system.

The master control unit comprises a master robot arm as described in the foregoing. The master control unit further comprises a cart.

The cart allows for transporting and/or repositioning of the master control unit, e.g. transporting the master control unit from a location to another, or repositioning the master control unit (and the master robot arm) to a desired position for surgery.

The master control unit provides a stable platform for the surgeon to reliably operate the master robot arm.

To allow for easy transportation and repositioning, the cart may comprise a plurality of wheels.

For instance, the cart may comprise four wheels.

Preferably, said wheels are swivel wheels.

Preferably, at least two of said wheels are provided with a brake.

Alternatively, all four wheels may be provided with a respective brake.

By acting on the brakes (e.g., by the user's foot), rotation of the wheels can be inhibited, so that the master control unit is maintained in place in a desired position, e.g. for surgery.

The wheels may have a diameter of approximately 125 mm.

Also, the wheels may be adapted to carry a load of approximately 130 kg.

The cart may further comprise a handlebar.

The handlebar allows the user to easily and safely moving/orienting the master control unit.

The handlebar can be conveniently arranged at the back side of the cart.

The handlebar can be formed by the cover itself or by an outer part of the system.

The cart may further comprise an armrest to increase the user's comfort during operation.

Tests have been conducted by the inventors to determine a convenient armrest design. The results advised that an ideal armrest would have a width of 80-100 mm, rounded edges, and layers of gel measuring 20 mm or 30 mm.

Ultimately, a possible armrest design has been defined, featuring a width of 73 mm with rounded edges and a 20 mm gel layer on top.

Nevertheless, other configurations for the armrest are possible according to the invention.

The cart may further comprise a pedal unit.

The pedal unit comprises at least one pedal.

At least one pedal is arranged on a platform, which forms part of the cart.

Preferably, the pedal unit comprises a first pedal and a second pedal.

The first and second pedals may have a binary function.

This ensures that two different scaling can be used during surgery.

Scaling can be controlled through dedicated software means.

Preferably, the pedal unit further comprises a microscope pedal.

The microscope pedal is also arranged on the platform.

In particular, a housing may be formed on the platform to accommodate the pedal.

At least one pedal is electrically connected to the cart.

Preferably, this connection is achieved by using a cable, which is integrated into the cart.

The cable further ensures that the pedal can only physically reach a predefined, limited range of motion.

The cart may further comprise a mounting interface.

The mounting interface allows for connection of the master robot arm to the cart.

Conveniently, the mounting interface can be a (3D-)printed part or a milled part or any other suitable produced and connected to an interface profile of the cart.

Fixation of the mounting interface to the interface profile can be implemented, e.g., by screwing.

The first distal joint element of the master robot arm can be arranged within said mounting interface.

The cart may further comprise a holding means configured for holding the master robot arm in a parking position.

This allows that the master robot arm remains in a safe, stable position during transport within a hospital facility.

The cart may further comprise a display means to display relevant information to the surgeon concerning a surgical, microsurgical or super-microsurgical procedure being carried out.

Preferably, information is displayed in real time.

A Graphical User Interface (GUI) may be displayed on said display means.

The GUI may be configured to show a current status of the robot system and provide certain option selections for the user.

Information displayed on the GUI shall be clearly visible for the surgeon during operation.

For instance, the GUI shall be arranged in the same fashion as a car dashboard.

Care should be taken to set a convenient angle for the display means to prevent reflections from ceiling lighting.

Also, the display means shall be located within arm's reach for the surgeon, to allow for easy selection of one or more commands/options included in the displayed GUI.

The display means may include a screen having a 7-inch diagonal size and a 16:9 ratio.

The screen may be horizontally-oriented to optimally display the required content.

The screen may be implemented with touch capabilities, allowing a user to select a displayed option or otherwise introduce one or more commands with greater ease.

The master control unit may also comprise a second master robot arm, serving as master supervisor.

The master control unit may be further implemented with a means providing the user with relevant information concerning operational state and errors of the robot system, preferably in real time.

Eventually, the invention provides a surgical, microsurgical or super-microsurgical robot system.

The robot system comprises a master control unit as described in the foregoing.

The robot system further comprises at least one, preferably twoslave robot arm(s).

The slave robot arm is actuated by a master robot arm, such as the master robot arm described in the foregoing.

In particular, the master robot arm actuates the slave robot arm according to a received user input (e.g. provided though a user interface such as a joystick), so that a surgical instrument arranged at the distal end of the slave robot arm is operated and manipulated accordingly.

As a non-limiting example, the slave robot arm can be adapted to perform anastomoses.

As a non-limiting example, the surgical instrument can be a hinged surgical instrument, e.g. a scissors-type or a forceps-type surgical instrument.

Still further, the robot system comprises a robot cart.

The robot cart allows transporting/positioning the robot system.

Preferably, the robot system has a maximum weight of 100 kg, so that it can be easily transported and maneuvered within a hospital facility.

In particular this maximum weight is a tradeoff between stability and a solid feeling and ease of maneuverability.

Further details and advantages of the present invention shall now be disclosed in connection with the drawings, where:
- **Fig. 1**: is a diagram illustrating the main sub-systems of a robot system for use in surgery, microsurgery or super-microsurgery according to the prior art, along with their main functions and interactions. Here, the robot system comprises three main sub-systems, namely: a master robot arm, a robot cart, and a slave robot arm;
- **Fig. 2**: is a perspective view of a master robot arm for a master control unit according to an embodiment of the present invention. Here, the master robot arm has 7 DOF;
- **Fig. 3**: shows a cart for use in a master control unit, according to an embodiment of the invention. The cart allows for transportation/positioning of the master control unit (and the master robot arm);
- **Fig. 4**: shows a detail of the armrest of the cart of **Fig. 3** in two different positions, namely: **a** non-retracted position; **b** retracted position;
- **Fig. 5**: shows a mounting interface for connecting a master robot arm, such as the master robot arm of **Fig. 2****,** to a cart, such as the cart of **Fig. 3****;**
- **Fig. 6**: is a diagram illustrating how a dome of a joystick (user interface) rotation lies within the workspace for the left master robot arm. Right robot arm is mirrored;
- **Fig. 7**: is a cross-section view of the first distal joint element of the master robot arm of **Fig. 2****;**
- **Fig. 8**: is a cross-section view of the second distal joint element of the master robot arm of **Fig. 2****;**
- **Fig. 9**: is a cross-section view of the first intermediate joint element of the master robot arm of **Fig. 2****;**
- **Fig. 10**: is a cross-section view of the second intermediate joint element of the master robot arm of **Fig. 2****;**
- **Fig. 11**: is a cross-section view of the first proximal joint element of the master robot arm of **Fig. 2****;**
- **Fig. 12**: is a detail view showing a clock spring structure mounted to the first proximal joint element and the second proximal joint element;
- **Fig. 13**: is a cross-section view of the second proximal joint element of the master robot arm of **Fig. 2****;**
- **Fig. 14**: is a cross-section view of a proximal arm element in the shape of a joystick, allowing the surgeon to actuate the master robot arm. The joystick is configured to perform an opening/closing movement, adding 1 DOF;
- **Figs. 15a-b**: are diagrams showing a cross-section view of the joystick of **Fig. 14** in different conditions, namely: **a.** open state and **b.** closed state;
- **Fig. 16**: shows a slider crank mechanism for use in the joystick of **Fig. 14****;**
- **Fig. 17**: is a free body diagram of the joystick of **Fig. 14****,** schematically illustrating how forces act on its components;
- **Fig. 18**: is a diagram showing an exemplary magnet stack for use in the joystick of **Fig. 14****;**
- **Figs. 19a-b**: are diagrams showing two different magnet solutions for the joystick of **Fig. 14****.** In particular: **a.** one magnet polarized in one direction with a 5-degree imperfection of the magnetic field. The first two images show simulated values, the third image shows measured values; **b.** a proposed magnet-stack with 3 opposed magnets and iron cores, 5-degree imperfection on the magnetic fields. The first two images show simulated values, the third image shows measured values;
- **Fig. 20**: is a diagram schematically illustrating positioning of the first distal joint element, the second distal joint element, and the first intermediate joint element of the master robot arm of **Fig. 2****;**
- **Fig. 21**: is a diagram showing a schematic overview of the master robot arm of **Fig. 2****,** including information for determining angle Alpha and Theta;
- **Fig. 22**: is a diagram showing a schematic overview of the master robot arm of **Fig. 2****,** including information for determining mass energy;
- **Fig. 23**: are diagrams showing simulation results regarding the amount of energy necessary to compensate for gravity for different joint lengths and different positions;
- **Fig. 24**: are diagrams schematically showing configurations that are needed to reach each corner of a surgical workspace;
- **Fig. 25**: are diagrams illustrating ranges of motions in a coordinate system for each joint for: **a.** a left master robot arm, and **b.** a right robot arm for a master control unit of a surgical, microsurgical or super-microsurgical robot system;
- **Fig. 26**: is a diagram illustrating a shaft and connected encoder. Tolerances for the encoder are indicated in the figure;
- **Fig. 27**: are diagrams illustrating angular delay and parameters triangle necessary to determine the angular delay;
- **Fig. 28**: are diagrams illustrating three different positions (identified as Pos1, Pos2 and Pos3, respectively) for which angular delay has been calculated;
- **Fig. 29**: is a diagram illustrating forces acting on a bearing adapted for use in the joint elements of the master robot arm of **Fig. 2****;**
- **Fig. 30**: are free body diagrams illustrating forces which have an influence on the axial and radial forces acting on the bearings in the first distal joint element;
- **Fig. 31**: are free body diagrams illustrating forces which have an influence on the axial and radial forces acting on the bearings in the second distal joint element;
- **Fig. 32**: is a diagram illustrating results of a simulation test focused on rest torque on joints of the gravity compensator of the second distal joint element;
- **Fig. 33**: are free body diagrams illustrating forces which have an influence on the axial and radial forces acting on the bearings in the first intermediate joint element;
- **Fig. 34**: is a diagram illustrating results of a simulation test focused on rest torque on joints of the gravity compensator the first intermediate joint element;
- **Fig. 35**: are free body diagrams illustrating forces which have an influence on the axial and radial forces acting on the bearings in the second intermediate joint element;
- **Fig. 36**: are free body diagrams illustrating forces which have an influence on the axial and radial forces acting on the bearings in the first proximal joint element;
- **Fig. 37**: are free body diagrams illustrating forces which have an influence on the axial and radial forces acting on the bearings in the second proximal joint element;
- **Fig. 38**: are diagrams showing analyzed deformation occurring in the cart of the master control unit. In particular: **a.** input model, with a point of force at location P and 3 different boundary conditions; **b.** results of directional deformation simulation for 250N; **c.** results of directional deformation simulation for 1000N;

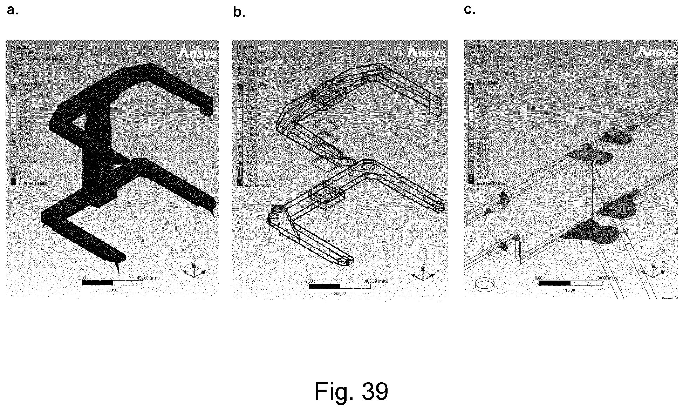

- **Fig. 39**: are diagrams showing equivalent stress simulation for the cart of the master control unit. In particular: **a.** results of equivalent stress simulation for 100N; **b.** results of equivalent stress simulation with a threshold of 235MPa; **c.** highest value of equivalent stress with a threshold of 235 MPa.

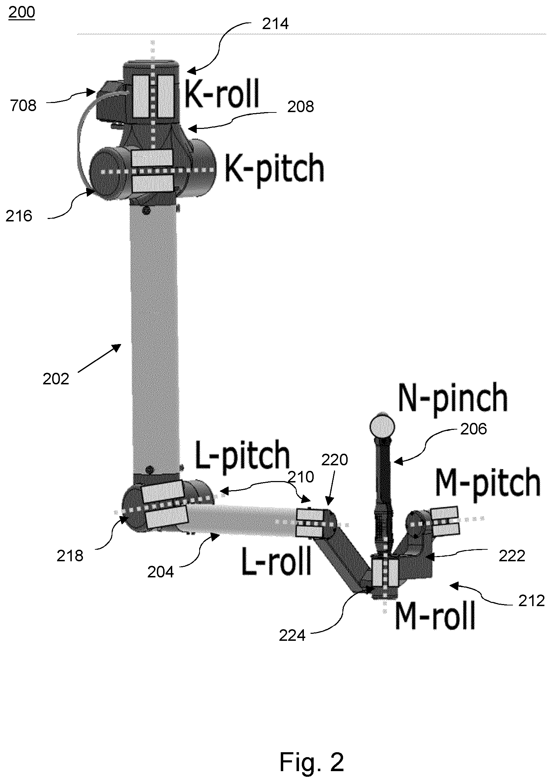

**Fig. 2** shows a master robot arm 200 according to an embodiment of the invention.

The master robot arm 200 is adapted for use in a master control unit 400 of a surgical, microsurgical or super-microsurgical robot system 100.

In general, surgical, microsurgical or super-microsurgical robot systems comprise three main sub-systems, namely a master control unit 400, a slave robot arm 500, and a robot cart 600.

An overview of these sub-systems 400, 500, 600 along with their main functions and interactions is provided in **Fig. 1****.**

As mentioned, in the context of the present application, the term "proximal" denotes a side which is closer to the user, e.g. a surgeon.

Similarly, the term "distal" denotes a side that is farther from the user.

In the present embodiment, as shown in **Fig. 2****,** the master robot arm 200 comprises a first, distal arm element 202, a second intermediate arm element 204, a third, proximal arm element 206, a first, distal joint 208, a second, intermediate joint 210, and a third, proximal joint 212.

The distal joint 208 comprises a first distal joint element 214 configured to perform a first roll movement, and a second distal joint element 216 configured to perform a first pitch movement (**Fig. 2**).

The intermediate joint 210 comprises a first intermediate joint element 218 configured to perform a second pitch movement, and a second intermediate joint element 220 configured to perform a second roll movement (**Fig. 2**).

The third, proximal joint 212 comprises a first proximal joint element 222 configured to perform a third pitch movement and a second proximal joint element 224 configured to perform a third roll movement (**Fig. 2**).

The features of the joint elements 214, 216, 218, 220, 222, 224 will be described in greater detail in the following.

The distal joint 208, the intermediate joint 210, and the proximal joint 212 provide the master robot arm 200 with six degrees of freedom (DOF).

That is, components of the master robot arm 200 can be orientated in different lateral and rotational directions, to provide a 6-DOF-input at the distal end of a slave robot arm 500, to operate and manipulate at least one surgical instrument connected thereto.

The proximal arm element 206 provides a user interface allowing a user to provide a user input for the master robot arm 200.

In the present embodiment, the proximal arm element 206 is in the shape of a joystick, serving as a hand interface for a user to provide a user input.

In particular, in the present embodiment, the joystick 206 comprises a pair of hinged levers 226, configured to perform an opening/closing movement with respect to one another (**Fig. 2**).

Accordingly, the master robot arm 200 is provided with an additional degree of freedom (DOF).

That is, the master robot arm 200 according to the present embodiment is characterized by seven degrees of freedom (DOF).

In particular, 6 DOF serve to operate and manipulate a surgical instrument (not shown) provided at the distal end of the slave robot arm 500.

The additional DOF serves to control an opening/closing/pinching operation of the surgical instrument, e.g. a scissors-type or a forceps-type surgical instrument.

The features of the joystick 206 will be described in greater detail in the following.

The master robot arm 200 of the invention has a reduced mechatronic complexity compared to known solutions.

In particular, the master robot arm 200 has a passive design, which does not include any active component such as motors and/or brakes.

This allows optimizing kinematics of the master robot arm 200, in turn resulting in enhanced precision and reliability during manipulation.

At least one of the joint elements 214, 216, 218, 220, 222, 224 can be provided with a respective encoder

The encoder allows sensing a position of a respective joint element.

The encoder can be a magnetic encoder.

Alternatively, the encoder can be an optical encoder.

For instance, in the present embodiment, the first distal joint element 214, and/or the second distal joint element 216, and/or the first intermediate joint element 218 may comprise an optical encoder.

Also, the second intermediate joint element 220, and/or the first proximal joint element 222, and/or the second proximal joint element 224 may comprise a magnetic encoder.

Advantageously, in the present embodiment, the distal joint 208 is characterized by the following ranges of motion:
first roll movement: +-inf, and/or
first pitch movement: -5° to +30° with respect to the first pitch rotation axis.

Advantageously, in the present embodiment, the intermediate joint 210 is characterized by the following ranges of motion:
second pitch movement: +62° to +118° with respect to the second pitch rotation axis, and/or
second roll movement: -80° to +80° with respect to the second roll rotation axis.

Advantageously, in the present embodiment, the proximal joint 212 is characterized by the following ranges of motion:
third pitch movement: -80° to +80° with respect to the third pitch rotation axis, and/or
third roll movement: +-inf.

In the present embodiment, the distal arm element 202 has a length between 250 mm and 400 mm, preferably of 350 mm.

Also, in the present embodiment, the intermediate arm element 204 has a length between 250 mm and 400 mm, preferably of 350 mm.

In the present embodiment, the master control device 200 comprises at least one mass gravity means.

This allows making the master control unit 400 (and the master robot arm 200) stable against gravitational components.

In particular, in the present embodiment, the at least one gravity compensation means comprises a first gravity compensation means, arranged in the second distal joint element 216, and a second gravity compensation means, arranged in the first intermediate joint element 218.

The second intermediate joint element 220 and the second proximal joint element 224 are balanced in a predefined pose.

In particular, this is achieved by tuning the center of mass properties in such a way that these joint elements 220, 224 have a stable pose in a predefined, desired orientation.

The master robot arm 200 is adapted for use in a master control unit 400 of a surgical, microsurgical or super-microsurgical robot system 100.

Accordingly, the invention further provides a master control unit 400, comprising the master robot arm 200 described in the foregoing.

The master control unit 400 further comprises a cart 700.

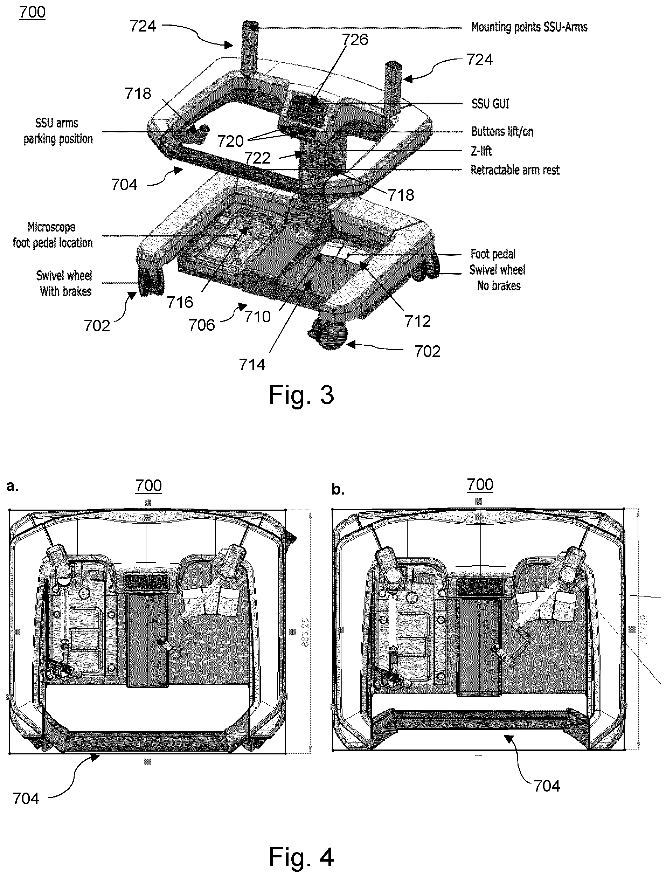

**Fig. 3** shows a cart 700 for use in the master control unit 400 of the invention.

The cart 700 allows transporting the master control unit 400 from one location to another within a hospital facility, and/or orienting the master control unit 400 (and the master robot arm 200) to a desired position for surgery.

For this purpose, the cart 700 is provided with a plurality of wheels 702, in particular swivel wheels 702.

In the configuration of **Fig. 3****,** the cart 700 has four swivel wheels 702.

In the present embodiment two of the wheels 702 are provided with a brake. However, different solutions are also possible according to the invention.

For instance, in an alternative configuration (now shown), each wheel 702 may be provided with a brake.

The master control unit 400 can be stabilized in a desired position by acting on the brakes (e.g., by the user's foot), thus inhibiting rotation of the wheels 702.

A handlebar is provided on the back side of the cart 700 to allow easily and safely moving/orienting the master control unit 400. The handlebar can be formed also in a possible embodiment by a part of the frame.

The cart 700 further comprises a pedal unit 706 (**Fig. 3**).

The pedal unit 706 comprises at least one pedal.

In anembodiment, the pedal unit 706 comprises a first pedal 710 and a second pedal 712, arranged on a platform 714. It is possible to also have this optional and

In the shown embodiment, the pedal unit 706 further comprises a microscope pedal.

The microscope pedal is arranged on the platform 714.

A housing 716 may be formed in the platform 714 for arrangement of the microscope pedal.

The cart 700 further comprises an armrest 704 (**Fig. 3**).

The armrest 704 allows providing comfort and support for the user's forearm.

The cart 700 further comprises a plurality of buttons 720, as shown in **Fig. 3****.**

A first button can be provided for powering the master control unit 400 on/off.

Other buttons 720 are provided for controlling a height adjustment mechanism 722 for adjusting a height of the cart 700 as desired.

The armrest 704 is configured to switch between a non-retracted position (**Fig. 4a**) and a retracted position (**Fig. 4b**).

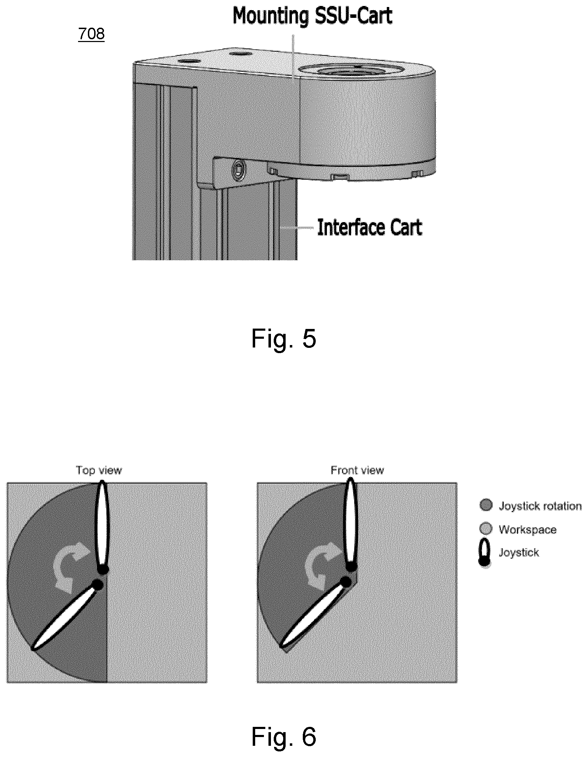

The cart 700 further comprises a mounting interface 708, as illustrated in detail in **Fig. 5****.**

In particular, the mounting interface 708 is configured for connection with mounting points 724 formed in the cart 700.

In the shown configuration, the cart 700 comprises two mounting points 724.

The mounting interface 708 is a milled or printed part configured for connection to an interface profile of the cart 700, to allow for connection of the master robot arm 200 to the cart 700.

As a non-limiting example, connection between the mounting interface 708 and the and a respective mounting point 724 of the cart 700 can be implemented by screwing.

In the shown embodiment, the cart further comprises a holding means 718 configured for holding the master robot arm 200 in a parking position.

In the shown embodiment, the cart 700 further comprises a Graphical User Interface (GUI) 726.

The GUI 726 may be configured to show a current status of the robot system and provide certain option selections (e.g. instrument selection) for the user. The master control unit 400 may further comprise a second master robot arm 200.

The present invention further provides a robot system 100 for use in surgical, microsurgical or super-microsurgical procedures.

The robot system 100 comprises a master control unit 400 as described in the foregoing.

Also, the robot system 100 comprises a slave robot arm 500.

The slave robot arm 500 is operated and manipulated through the master robot arm 200 (**Fig. 2**) of the master control unit 400, based on an input provided by the user.

In the present embodiment, as described in the foregoing, the master robot arm 200 provides a 6-DOF-input for manipulating a surgical instrument (not shown) provided at the distal end of the slave robot arm 500, and an additional DOF for an opening/closing/pinching operation of the instrument, implemented through the joystick 206.

As a non-limiting example, the slave robot arm 500 can be adapted to perform anastomoses.

As a non-limiting example, the surgical instrument can be hinged surgical instrument, e.g. a scissors-type or a forceps-type surgical instrument.

Still further, the robot system 100 comprises a robot cart 600.

The robot cart 600 allows for transportation/positioning of the robot system 100.

The robot cart 600 further allows fixating the robot system 100 in a desired position for surgery.

### Kinematics of the master robot arm

To perform surgical, microsurgical or super-microsurgical procedures, the master robot arm must be able to be manipulated with a 160x160x160 mm cubic workspace, offering enough dexterity within this workspace. A user interface, in particular a joystick, must be able to be orientated within a halve dome.

To this end, the joystick is configured to be orientated to at least cover a 135° cone around the Z-axis in the defined workspace.

A visual representation of how the dome of the joystick rotation lies within the workspace for a left master robot arm of a master unit is shown in **Fig. 6****.**

The right arm is mirrored.

### First distal joint element

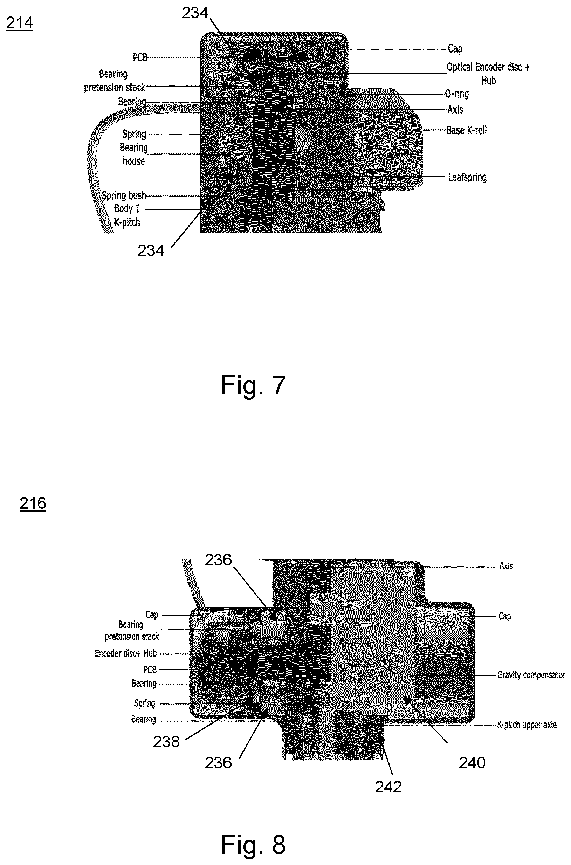

**Fig. 7** provides a cross-section view of the first distal joint element 214 of the master robot arm 200.

The first distal joint element 214 performs a first roll movement.

The motion of first distal joint element 214 ensures one of the translational degrees of freedom.

To enable the rotation of first distal joint element 214, an O arrangement of two bearings 234, in particular an upper bearing 234 and a lower bearing 234, is implemented.

This arrangement is within the base of the first distal joint element 214.

The bearings 234 are placed under pretension to eliminate internal clearance and to help with the alignment.

A leaf spring is further provided to ensure radial clearance free mounting.

The axis of the distal joint element 214 is the same as that of the second distal joint element 216.

There is a stroke limiter in the distal joint element 214.

Stroke limitations for distal joint element 214 range from 0° to 45.

The upper bearing 234 and a bearing pretension stack carry the load of the entire robot arm 200.

### Second distal joint element

**Fig. 8** provides a cross-section view of the second distal joint element 216 of the master robot arm 200.

The second distal joint element 216 performs a first pitch movement.

The motion of the second distal joint element 216 is a rotational motion which ensures one of the position degrees of freedom.

The range of this motion is limited to 45°.

This limitation is accomplished by using a pin which is adapted to engage with a slot, this slot being limited to a range of 45.5°.

The rotation of the second distal joint element 216 is achieved through an O arrangement of two bearings 236.

This arrangement is pretensioned by means of a spring 238.

A leaf spring is also provided to ensure radial clearance free mounting.

The second distal joint element 216 is provided with a gravity compensation means 240.

The second distal joint element 216 comprises an upper axle 242 and a lower axle 246.

### First intermediate joint element

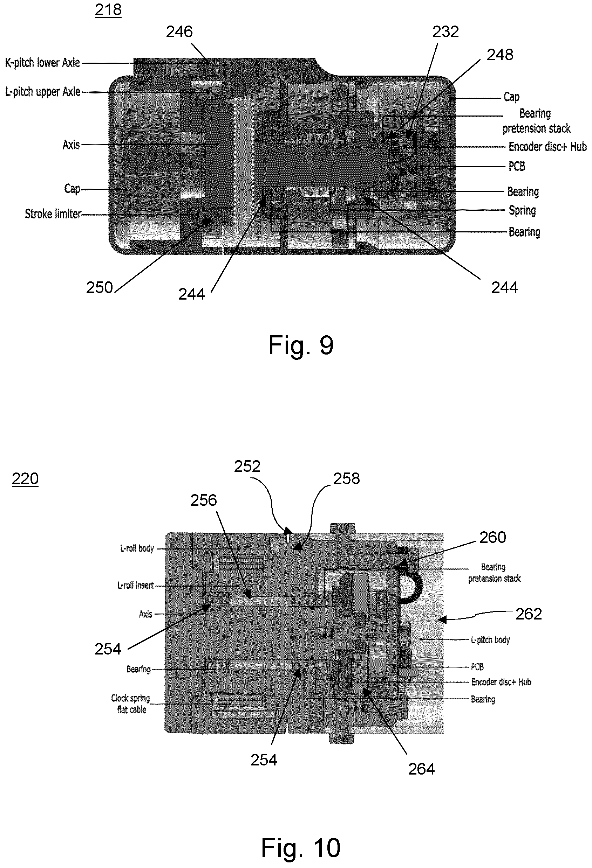

**Fig. 9** provides a cross-section view of the first intermediate joint element 218 of the master robot arm 200.

The first intermediate joint element 218 performs a second pitch movement.

The motion of the first intermediate joint element 218 ensures one of the position degrees of freedom.

The range of this motion is limited to 41°.

Limitations is accomplished by way of a stroke limiter 250.

In particular, limitation is embedded in the gravity compensation means of the first intermediate joint element 218 (further details in the following). The rotation of the first intermediate joint element 218 is achieved through an O arrangement of two bearings 244.

This arrangement is provided within the lower axle 246 of the first intermediate joint element 218.

The bearings 244 are locked up by means of a pretension stack 248.

A leaf spring is also provided well to ensure radial clearance free mounting.

### Second intermediate joint element

**Fig. 10** provides a cross-section view of the second intermediate joint element 220 of the master robot arm 200.

The second intermediate joint element 220 performs a second roll movement.

The motion of second intermediate joint element 220 ensures one of the rotational degrees of freedom.

To enable rotation of the second intermediate joint element 220, an O arrangement of two bearings 254 with a spacer 256 has been implemented.

This arrangement 254, 256 is provided within a housing 258, formed in an insert 252 of first proximal joint element 222.

In this arrangement an O-ring serves as a spring to compensate for the radial clearance, and a wave spring is used to pretension a bearing stack 260.

The insert 252 is mounted to the body 262 of the first intermediate joint element 218.

The rotation of the second intermediate joint element 218 is limited to -125 to 35 degrees by means of a mechanical limiter.

There is an optical encoder PCB 264 which is mounted to the body 262 of the first intermediate joint element 218.

A clock spring structure is provided to ensure that a daisy chain of encoders in the robot arm 202 may continue.

### First proximal joint element

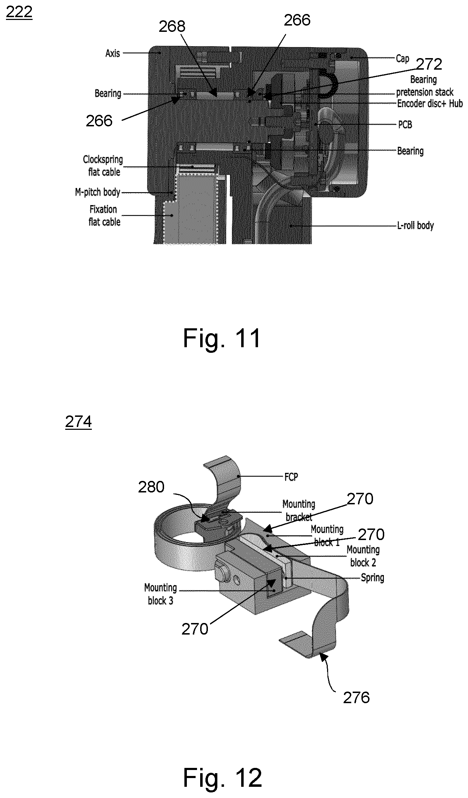

**Fig. 11** provides a cross-section view of the first proximal joint element 222 of the master robot arm 200.

The first proximal joint element 222 performs a third pitch movement.

The motion of the first proximal joint element 222 ensures one of the rotational degrees of freedom.

To enable rotation of the first proximal joint element 222, an O arrangement of two bearings 266 with a spacer 268 has been implemented.

This arrangement 266, 268 is provided within the housing 258 of the of the first proximal joint element 222.

In this arrangement, an O-ring serves as as a spring to compensate for the radial clearance and a wave spring will pretensions the bearing stack 272.

The rotation of this stroke is limited by a mechanical limiter to 174 degrees.

The housing 258 of the of the first proximal joint element 222 and a housing of the second proximal joint element 224 are fitted upon each other.

An optical encoder is provided in the first proximal joint element 222 for read out of an angle of this joint element 222.

Here, the provision of an optical encoder is rendered possible by the fact that a decision has been made not to use a slip ring, but rather use a clock spring structure 274 with a flat cable 276, as illustrated in **Fig. 12****.**

This structure 274 also allowed to obtain a more compact design.

The structure 274 is mounted to a body of the second intermediate joint element 220 through a mounting block 270 (including three parts that are respectively identified with 1, 2, 3 in **Fig. 12**), and to a housing 258 of the first intermediate joint element 218 though a mounting bracket 280.

This ensures that rotation of the cable 276 only happens in the clock spring shape. An flexible printed circuit (FPC )is glued upon the mounting bracket 280.

The mounting blocks 270 together with springs ensure that the FPC is fixated to the mounting blocks 270 and the mounting blocks 270 are mounted to the body of the second intermediate joint element 220.

If a housing of the second intermediate joint element 220 starts to rotate, the mounting blocks 270 will also rotate, which will tighten the pitch of the clock spring structure and enable rotation of the joint element while still having data transfer.

### Second proximal joint element

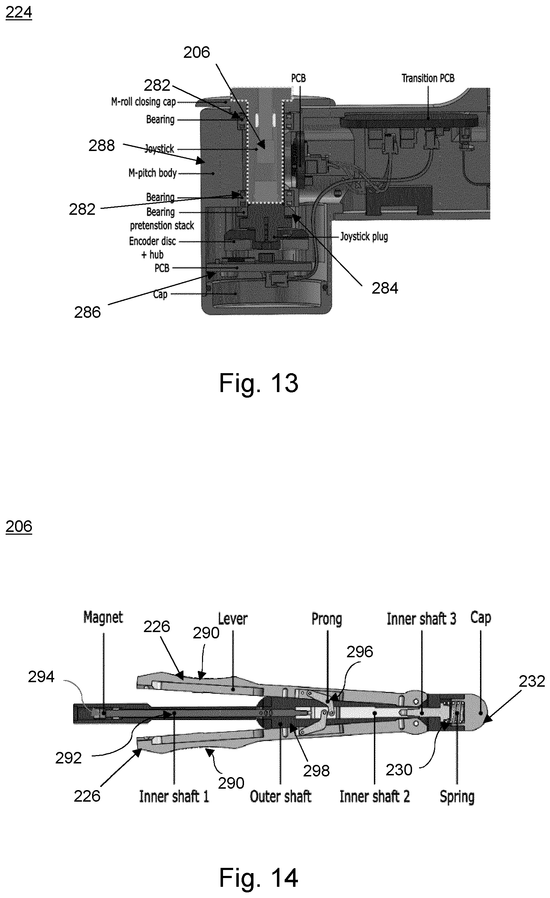

**Fig. 13** provides a cross-section view of the second proximal joint element 224 of the master robot arm 200.

The second proximal joint element 224 performs a third roll movement.

The second proximal joint element 224 allows rotation of the entire joystick 206. All components of the joystick 206 will thus rotate.

The rotation is facilitated by two bearings 282 provided in the housing of the second proximal joint element 224.

The bearings 282 are in an O arrangement.

In this arrangement, an O-ring serves as a spring to compensate for the radial clearance and a wave spring pretension a bearing stack 284.

An optical encoder PCB 286 is mounted to a body 288 of the first proximal joint element 222.

### Joystick

**Fig. 13** provides a cross-section view of the is a cross-section view of the proximal arm element, here in the shape of a joystick 206.

The joystick 206 is the part that the surgeon holds during surgery.

In particular, when surgery is performed, the surgeon holds the joystick 206 through the levers 226.

The levers 226 are implemented with ergonomic indicators to facilitate correct gripping of the joystick 206.

The joystick 206 is designed in such a manner that it has a 3-point support, namely the thumb, the index finger and the thenar web space.

The levers 226 of the joystick 206 have slits 290 formed therein to indicate where the user shall place his/her fingers to properly hold and manipulate the joystick 206.

As mentioned, the levers 226 of the joystick 206 are configured to perform a motion of opening/closing with respect to one another.

Motion of the levers 226 is translated into a linear motion of an inner shaft 292.

The inner shaft 292 consists of three different parts, denoted with 1, 2 3 in **Fig. 14****.**

The inner shaft 292 houses a magnet 294, and is configured to receive linear input from prongs 296.

The inner shaft 292 translates the lever 226 movement into a corresponding magnet 294 motion.

The magnet 294 serves as input for a linear sensor to detect a position of the inner axis of the joystick and determine closure of the joystick 206.

The prong 296 translates a motion of the levers 226 into a linear motion of the inner shaft 292.

The joystick 206 further comprises an outer shaft 298.

The outer shaft 298 facilitates rotation of the second proximal joint element 224 and acts as a fixed world for the levers 226.

The outer shaft 298 is inserted to the bearings 282 of the second proximal joint element 224.

To ensure that the joystick 206 automatically opens and gives some force feedback, a spring 230, preferably a compression spring, is arranged between the inner shaft 292 and a cap 232.

The outer shaft 298 serves as a mounting location for the cap 232.

The cap 232 is added to the joystick 206 to ensure that the spring 230 can be assembled and has a fixed world.

When closed, the joystick 206 has a diameter of approximately 16 mm while, when open, the joystick 206 has a diameter of approximately 28 mm.

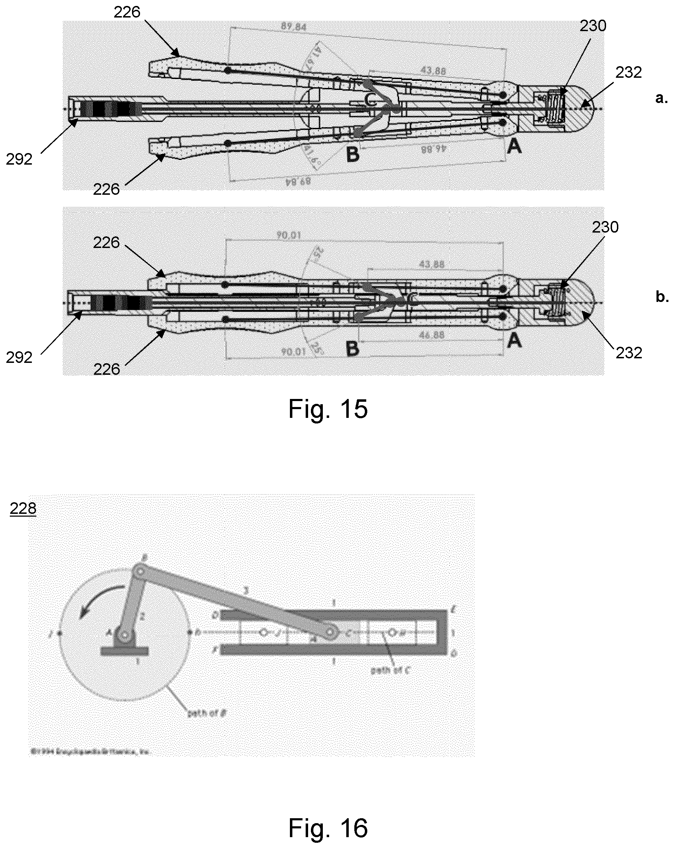

The joystick 206 is configured to perform a pinching movement, where the levers 226 are brought from an open state (**Fig. 15a**) to a closed state (**Fig. 15b**).

The rotating motion at the end of the lever 226 is translated into a linear motion of the inner shaft 292.

The lever 226 is mounted to the outer shaft 298 and rotates compared to the outer shaft 298.

The translation of the lever motion to the linear motion is achieved by adding a linkage mechanism 224 (best shown in **Fig. 16**) in the top portion of the joystick 206.

One side of the linkage mechanism 228 will follow the circular path of the lever 226, while the other side will create a linear movement.

By having the linkage mechanism 228 on both levers 226 and on one linear axis, the design ensures that when one lever 226 moves, the other lever 226 will follow.

This is not entirely symmetrical since the rotational point of the levers 226 is not at the same location. However, this cannot be felt by the user while manipulating the joystick 206.

The inner shaft 292 has a displacement of approximately 1.8 mm.

This inner shaft 292 is connected to the spring 230, which ensures that the joystick 206 tend to stay open or automatically re-open after closing, and further provides some haptic feedback during use.

This spring 230 preferably has a spring rate of 5.476 N/mm and is pretensioned with 3.5 mm.

This ensures a sufficient pinching force for the joystick 206.

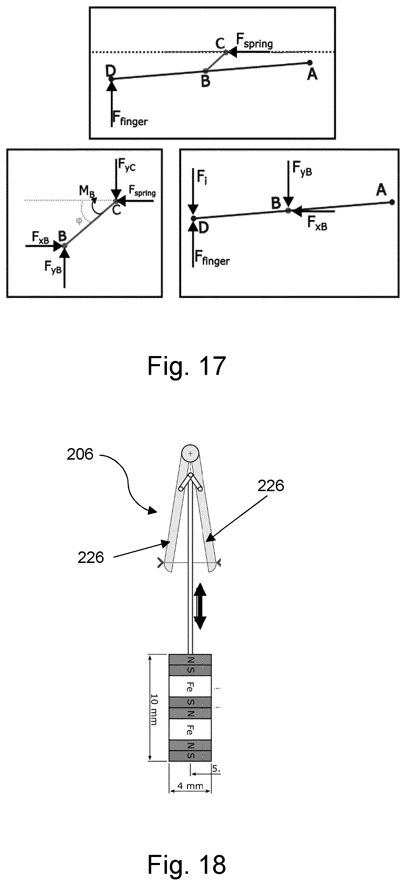

**Fig. 17** shows a free body diagram of the joystick 206, illustrating how forces act on joystick components and are related to each other.

Since the joystick components do not translate but only rotate, the following formulas can be obtained. ${F}_{spring} = {F}_{xB}$ ${F}_{yC} = {F}_{yB}$ ${F}_{yB} ⋅ cos \left(ϕ\right) - {F}_{xB} ⋅ sin \left(ϕ\right) = 0$ ${F}_{yB} = {F}_{xB} ⋅ tan \left(ϕ\right)$ ${F}_{finger} = {F}_{i} = {F}_{yB} ⋅ \frac{AB}{AD}$ ${F}_{finger} = \frac{{F}_{spring} ⋅ tan \left(ϕ\right) ⋅ AB}{AD}$

Calculated parameters and results regarding the pinching force of the joystick 206 are shown in **Table 1.**

### Magnetic encoder

A magnetic encoder is used to measure a location of the inner shaft 292 of the joystick 206.

This is mostly because a magnet field is homogeneous and therefore the rotation of the second proximal joint element 224 can be decoupled from pinching.

Initially, a simple magnet configuration was chosen by the inventors.

While the simulations appeared to be functioning, the testing results did not align.

This discrepancy was due to an imperfect orientation of the magnetic field, caused by the manufacturing process of the magnet resulting in an average angular deviation of 5 degrees, which was not considered.

This angular deviation showed itself in the data by having fluctuating values when rotating the joystick 206.

Once this deviation was accounted for, simulations corroborated the testing results. Based on this observation, a different approach was required.

Multiple simulations with various configurations were considered, and the optimal solution involved using multiple opposing magnets with iron cores arranged alternately.

This configuration is shown in **Fig. 18****.**

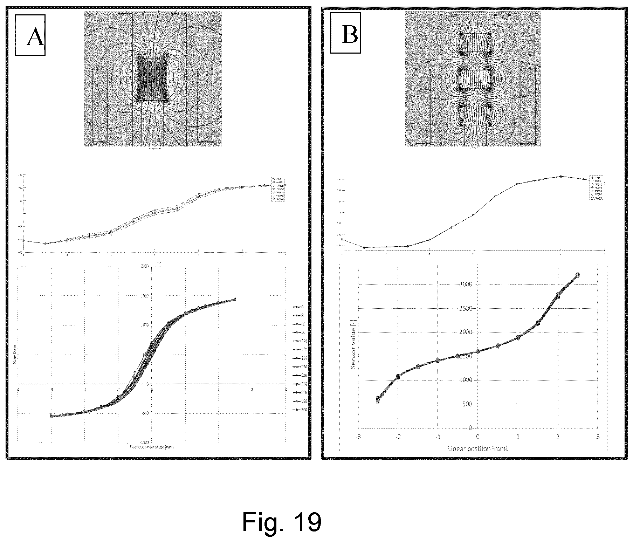

This configuration demonstrated consistent results in both simulations and real-world testing, as shown in **Fig. 19****.**

The setup that is used within the joystick 206 is a magnet stack of alternating magnets and iron cores (**Fig. 18**).

Each component is 2 mm thick and has a diameter of 4 mm.

The magnets have opposing fields.

The displacement of the inner shaft 292 of the joystick 206 is 1.8 mm and it is preferable that a sensor is located in a linear part of the measurement range. In particular, the sensor is placed so as to perform measurement in the range of -1.8 mm to 0 mm.

Preferably, the used encoder is capable of reaching a resolution of 0.98 µm when moving at speeds up to 8000 mm/s. The maximum velocity that is defined for the pinching movement is 23 mm/s and therefore the resolution for this application is 0.98 µm.

### Gravity compensation

Gravity shall be compensated for the position joint elements, namely the first distal joint element 214, the second distal joint element 216 and the first intermediate joint element 218, and settled to a natural point for the orientation joint elements, namely the second intermediate joint element 220, the first proximal joint element 222, and the second proximal joint element 224.

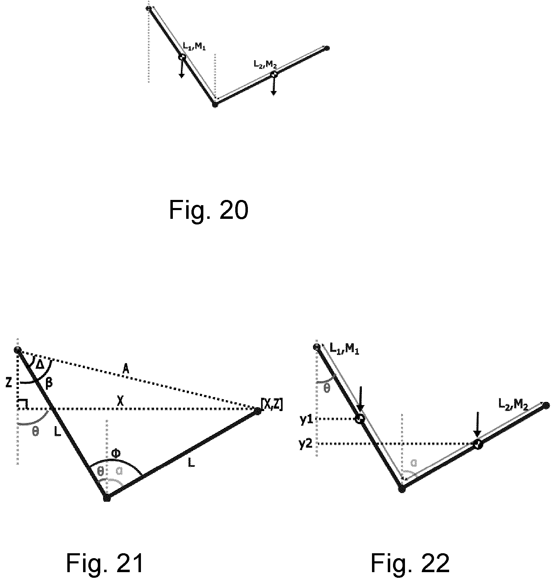

As shown in **Fig. 20****,** the first distal joint element 214 is not influenced by gravity.

Therefore, only the second distal joint element 216 and the first intermediate joint element 218 shall be gravity compensated.

### Length definition for the master robot arm

In order to determine an ideal length for the robot arm, two different aspects shall be considered, namely the total mass energy of the robot arm and the reach.

It is determined that the robot arm is mounted on point [0,0] and the tip of the robot arm has to reach a certain location in an array [X,Z].

Based on these inputs it is possible to calculate the angle Θ and α, which are used to calculate the mass energy.

The entire chain of calculations, referring to the scenarios shown in **Figs. 21-22****,** are shown in the following:
Configuration based on **Fig. 21****:** $A = \sqrt{{X}^{2} + {Z}^{2}}$ $ϕ = {cos}^{- 1} \left(\frac{{A}^{2} - 2 {L}^{2}}{- 2 {L}^{2}}\right)$ $β = {tan}^{- 1} \left(\frac{\left|X\right|}{\left|Z\right|}\right)$ $Δ = \frac{π - ϕ}{2}$ $Θ = β - Δ$ $α = ϕ - Θ$

Configuration based on **Fig. 22****:** ${y}_{1} = \frac{1}{2} {L}_{1} ⋅ cos θ$ ${y}_{1} = {L}_{1} ⋅ cosθ - \frac{1}{2} {L}_{2} ⋅ cos α$ ${E}_{massK - pitch} = \left(\frac{1}{2}{M}_{1}+{M}_{2}\right) ⋅ g ⋅ L ⋅ cosθ$ $+ \frac{1}{2} {M}_{2} ⋅ g ⋅ L ⋅ cos α$ ${E}_{massL - pitch} = {M}_{2} ⋅ g ⋅ L ⋅ cos α$

In the ideal situation the angles would behave as linearly as possible while having the closest mass energy to zero as possible.

Furthermore, the total size is also a factor which is considered.

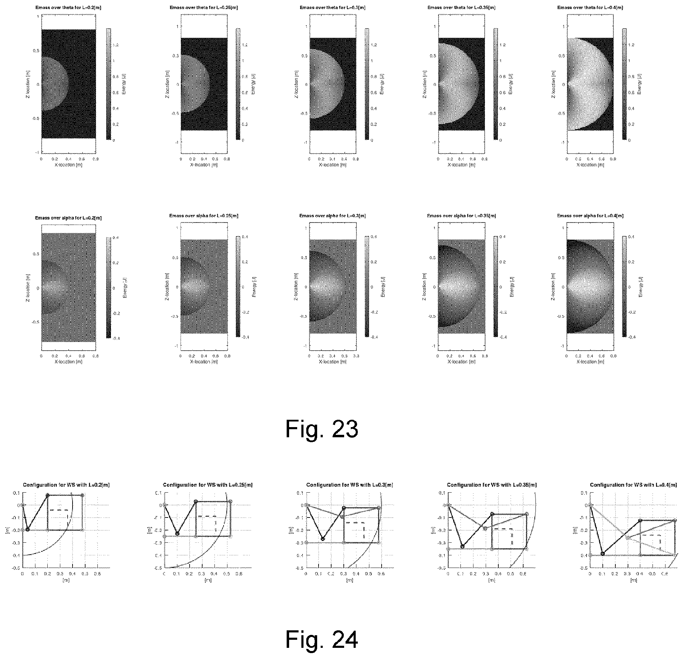

**Fig. 23** shows simulation test results of the amount of energy necessary to compensate for gravity for different joint lengths for different positions.

**Fig. 24** shows configurations that are needed to reach each corner of a surgical workspace.

For the simulation shown in **Fig 24****,** the location of a box (160x 160 mm) has been defined in such a manner that when Θ is 0° and φ is 90°, it can reach the bottom left corner of the workspace.

To ensure that the longest path in the cube can be reached, the box is defined as $\sqrt{3}$. 160 mm.

A general note is that the longer the arm length, the smaller the angular displacement for a given linear motion, reducing the curvature and making the motion feel more linear.

Mass energy calculations have been performed for M1= 0.2 kg, M2=0.25 kg and for L1=L2 ranging from 200 mm to 400 mm.

The results for different arm lengths based on the above calculations and this further input is shown in **Fig. 23****.**

The larger the linearity of the angles, the larger the mass energy that must be compensated and the larger the total size of the robot arm.

Therefore, it is a trade-off in size and mass energy versus linearity.

Based on the configurations of the workspace, possible working solutions for the arm length could be 250 mm, 300 mm, 350 mm, or 400 mm.

It is expected that length of 350 mm could represent a good trade off in size and mass energy versus linearity.

### Mechanical limits

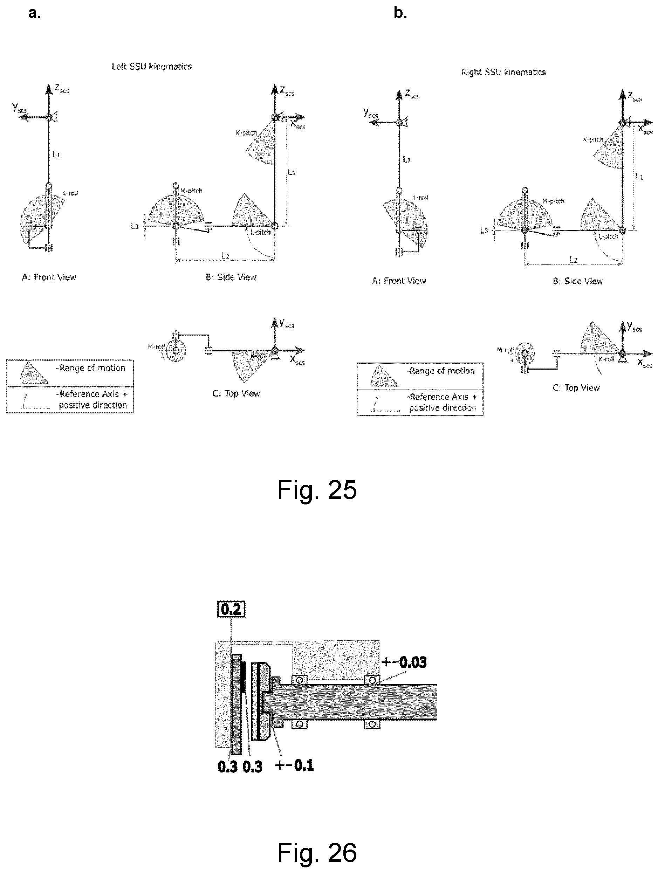

**Fig. 25** shows the mechanical limits for each joint element of the master robot arm.

In particular, mechanical limits are calculated for a master unit including a left robot arm (**Fig. 25a**) and a right robot arm (**Fig. 25b**).

A detailed overview of the ranges for each joint element of the left and right master robot arms is provided in **Table 2.**

### Angular error due to mechanical misalignment

The tolerance analysis considers the placement of an encoder input on an axis and the entire chain leading to chip's placement on a PCB.

These values are shown in **Fig. 26****.**

Based on this analysis, parallel misalignment tolerance can be calculated as follows: $Tolerance = 0.1 + \sqrt{{0.015}^{2} + {0.1}^{2}} + 0.15 + 0.15 = 0.5 \left[mm\right]$

To calculate the angular delay, several steps are required.

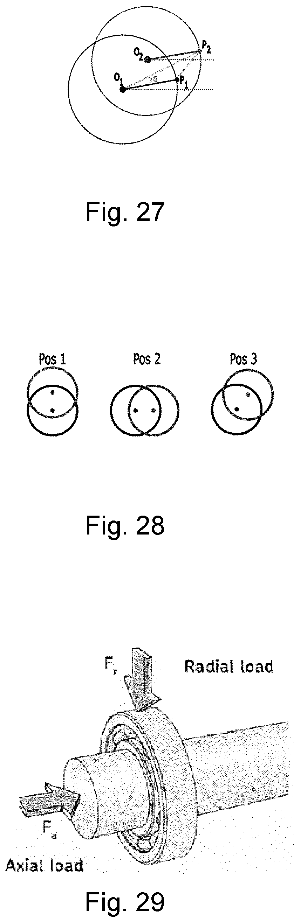

**Fig. 27** illustrates the parameters used for the calculation.

The distance between the two origins represents the tolerance, which was determined to be 0.5 mm.

The angle of the position on the circle relative to the origin (ϕ) is the same for both circles.

O1 is assigned [0,0]. ${P}_{2} = \left(\begin{matrix}{O}_{2 x} + r ⋅ cos \left(ϕ\right) \\ {O}_{2 y} + r ⋅ sin \left(ϕ\right)\end{matrix}\right)$ ${O}_{1} {P}_{2} = \sqrt{{P}_{2 x}^{2} + {P}_{2 y}^{2}}$ ${P}_{1} {P}_{2} = \sqrt{{\left({P}_{2 x}-{P}_{1 x}\right)}^{∧} 2 + {\left({P}_{2 y}-{P}_{1 y}\right)}^{∧} 2}$ $α = {cos}^{- 1} \left(\frac{{O}_{1} {P}_{2}^{2} + {r}^{2} - {P}_{1} {P}_{2}^{2}}{2 ⋅ {O}_{1} {P}_{2} ⋅ {O}_{1} {P}_{1}}\right)$

Tests have been carried out using an Aura^{™} 18 encoder.

In particular, angular delay has been calculated for different positions of parallel misalignment, which are illustrated in **Fig. 28** as Pos 1-3.

The maximum angular delay for each different position of **Fig. 28** is illustrated in **Table 3.**

### Encoder resolution

The master robot arm shall be able to measure its tip position (x-, y- and z-direction) with an accuracy of at least 50 µm.

This budget is distributed equally over the x-, y- and z-direction.

Therefore, the total budget can be thought of as the diagonal of a cube.

To calculate the diagonal of a cube the following formula can be used: ${D}_{cube} = \sqrt{{x}^{2} + {y}^{2} + {z}^{2}}$

Since the budget is distributed equally in all directions, the formula can be rewritten to: ${D}_{cube} = \sqrt{3 {x}^{2}} = x √ 3$

Therefore, the budget per joint element can be determined by dividing the total budget (diagonal of the cube) by √3.

This results in having a budget of 28.9 µm.

Furthermore, the master robot arm shall be able to measure its tip orientation (Rx, Ry and Rz) with an accuracy of at least 0.1 mrad.

The same concept as before is true, the budget per joint can be calculated by dividing the budget by √3.

This results in having a budget of 0.06 mrad.

To be able to determine this, it is first necessary to determine the moment arm lengths of the first three joint elements.

Maximum arm length of for first distal joint element: ${a}_{Kroll} \left[mm\right] = L ⋅ sin \left(45\right) + L = 648$

Maximum arm length of for the second distal joint element: ${a}_{Kpitch} \left[mm\right] = \sqrt{{a}_{Kroll}^{2} + {\left(L⋅cos\left(45\right)\right)}^{2}} = 694$

The moment arm length of the first intermediate joint element is the length of the master robot arm itself, here set as 350 mm.

The smallest angle that the encoder can read ( $\frac{2 π}{{2}^{# bit}}$) is so small, that it is possible to use a linearized function instead of the cosine rule for the accuracy of the position joints. ${accuracy}_{pos} \left[μm\right] = a ⋅ ϕ$ ${accuracy}_{ori} \left[rad\right] = \frac{2 π}{{2}^{# bit}}$

Based on these principles the results for the translational joints can be seen in **Table 4** and for the orientational joints in **Table 5.**

The accuracy of all joint elements falls within the allocated budget.

### Bearing calculations (for each joint element of the master robot arm)

To optimize the performance of the bearings and prevent deformation, the bearings that are used in the robot arm are pretensioned.

An appropriate spring and, in turn, a necessary pretension force, are determined through calculations that based on the momentum of the robot arm.

To fully exploit the basic load rating of the bearings, it is recommended to ensure that the forces are balanced, with equal magnitudes in both the radial and axial directions. For these calculations the human will not be in the loop.

Therefore, the acting forces are mainly caused by springs and gravity.

By dividing the basic static load rating (C) by the equivalent bearing load (P) a safety factor for the bearing can be calculated.

The basic static load rating is a known value of the bearing.

The bearing load can be calculated using the following formulas. $P = \left\{\begin{matrix}X ⋅ Fr + Y ⋅ Fa , & \frac{Fa}{Fr} \leq e \\ Fr , & \frac{Fa}{Fr} > e\end{matrix}\right.$ Where Fr is the radial load in kN, Fa is the axial load in kN, X and Y are load factors (these are standard table values for equivalent load bearings) and e is the critical ratio (value from the same table).

**Fig. 29** schematically shows the difference between axial and radial load acting on a suitable for use in the joint elements of the master robot arm according to the invention.

### Bearing calculations for the first distal joint element

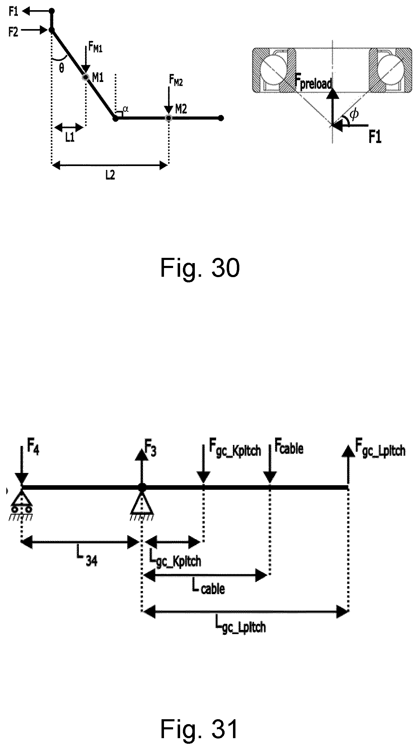

**Fig. 30** shows a free body diagram of forces which have an influence on the axial and radial forces acting on the bearings in the first distal joint element.

In the free-body diagram of **Fig. 30****,** most of the acting forces are due to the weight of robot arm itself.

M1 represents the center of mass of the first link, while M2 represents the center of mass of the second link, including the joystick.

The bearings in the first distal joint element experience the greatest impact when the angle (Θ) is 45° and the angle (α) is 90°.

To calculate the radial load on the bearings, a moment analysis must be performed. To this end, the following given values have been assumed.

M1 has a weight of 0.73 kg, with its center of mass (Lm1) located at 180 mm measured along the link.

M2 has a weight of 0.525 kg, with its center of mass (Lm2) located at 239 mm measured along the link.

The last given value is the length of the arm (La1) which is 350mm.

Also, it is assumed that the contact angle for the bearings is 10 deg (*ϕ*). ${L}_{1} \left[mm\right] = {L}_{m 1} ⋅ sin \left(Θ\right) = 180 ⋅ sin \left(45\right) = 127.3$ ${L}_{2} \left[mm\right] = {L}_{m 2} + {L}_{a 1} ⋅ sin \left(Θ\right) = 239 + 350 ⋅ sin \left(45\right) = 486.5$ $F 1 \left[N\right] = F 2 = \frac{{F}_{m 1} ⋅ {L}_{1} + {F}_{m 2} ⋅ {L}_{2}}{{L}_{12}} = \frac{0.73 ⋅ 9.81 ⋅ 127.3 + 0.525 ⋅ 9.81 ⋅ 486.5}{29.8} = 114.7$ ${F}_{preload} \left[N\right] = F 1 ⋅ tan \left(ϕ\right) = 114.7 ⋅ tan \left(10\right) = 20.2$

The minimum preload of the bearings, based on calculations, is determined to be 20.2 N.

In the final design, the preload was set at 22 N.

Furthermore, the first bearing carries the load of the entire arm.

Therefore, Fa=22+(0.73+0.525)*9.81= 34.3 N.

The forces that are calculated are utilized to evaluate whether the equivalent load provides a big enough safety margin compared to the basic static load rating, ensuring the bearing's suitability for this application (**Table 6**).

### Bearing calculations for the second distal joint element

**Fig. 31** shows a free body diagram of forces which have an influence on the axial and radial forces acting on the bearings in the second distal joint element.

In the free-body diagram of **Fig. 31****,** the bearings are represented with one modeled as a pin and the other as a roller.

This distinction is because one bearing can still move due to being mounted on leaf springs.

Based on simulations of the gravity compensator which is provided in this joint element, the maximum forces acting on the second distal joint element and the first intermediate joint element have been determined.

Second distal joint element: a maximum force of 177 N acts at a distance of 14.5 mm.

First intermediate joint element: a maximum force of 67 N acts at a distance of 47.7 mm.

Additionally, forces caused by the steel cables connecting said joint elements in the gravity compensator must be considered. These two cables are pretensioned to 50 N, and the resulting force acts at 27.2 mm. It is assumed that the contact angle for the bearings is 10 °(*ϕ*). $\begin{matrix}{F}_{4} \left[N\right] = \frac{{F}_{gcKpitch} ⋅ {L}_{gcKpitch} + {F}_{cable} ⋅ {L}_{cable} - {F}_{gcLpitch} ⋅ {L}_{gcLpitch}}{{L}_{34}} = \frac{177 ⋅ 14.5 + 100 ⋅ 27.2 - 67 ⋅ 47.7}{27} = 77.5 \\ {F}_{3} \left[N\right] = {F}_{4} + {F}_{gcKpitch} + {F}_{cable} - {F}_{gcLpitch} = 77.5 + 177 + 100 - 67 = 287.5 \\ {F}_{preload} \left[N\right] = {F}_{3} ⋅ tan \left(ϕ\right) = 287.5 ⋅ tan \left(10\right) = 51\end{matrix}$

The minimum preload of the bearings, based on calculations, is determined to be 51 N.

In the final design, the preload was set at 60 N.

The forces that are calculated are now utilized to evaluate whether the equivalent load provides a big enough safety margin compared to the basic static load rating, ensuring the bearing's suitability for this application (**Table 7**).

As mentioned, the second distal joint element shall gravity compensated.

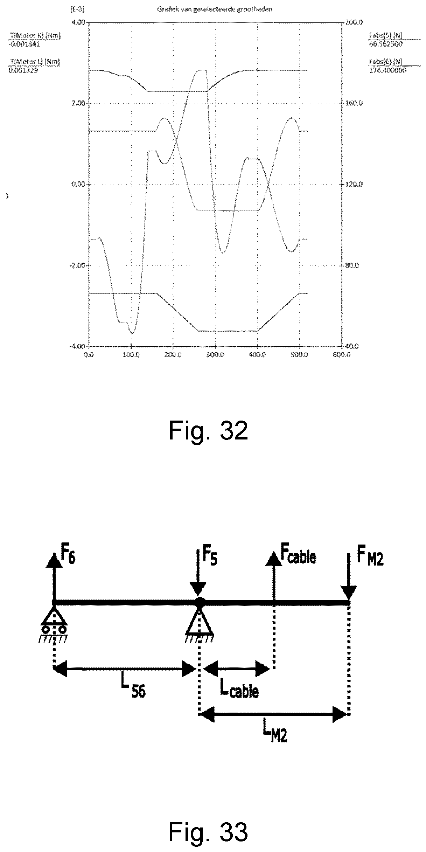

**Fig. 32** shows the results of a SAM simulation focused on the gravity compensator of this joint element.

These result show that the rest torque on the second distal joint element is never more than 4 mNm.

### Bearing calculations for the first intermediate joint element

**Fig. 33** shows a free body diagram of forces which have an influence on the axial and radial forces acting on the bearings in the first intermediate joint element.

In the free-body diagram of **Fig. 33****,** the bearings are represented with one modeled as a pin and the other as a roller.

This distinction is because one bearing can still move due to being mounted on leaf springs.

The forces causing an axial and or radial load on the joystick are the mass of the second link and gimble and the steel cables connecting the second distal joint element and the first intermediate joint element in the gravity compensator.

The mass is 0.525 kg acting at a distance of 14.5 mm, the two cables are pretensioned to 2 × 50 N, and the resulting force acts at 7.5 mm.

It is assumed that the contact angle for the bearings is 10 deg (*ϕ*). ${F}_{6} \left[N\right] = \frac{- {F}_{M 2} ⋅ {L}_{M 2} + {F}_{cable} ⋅ {L}_{cable}}{{L}_{56}} = \frac{- 0.525 ⋅ 9.81 ⋅ 14 + 100 ⋅ 7.5}{25} = 29.8$ ${F}_{5} \left[N\right] = - {F}_{M 2} + {F}_{cable} + {F}_{6} = - 0.525 ⋅ 9.81 + 100 + 29.8 = 124.6$ ${F}_{preload} \left[N\right] = {F}_{5} ⋅ tan \left(ϕ\right) = 124.6 ⋅ tan \left(10\right) = 22.0$

The minimum preload of the bearings, based on calculations, is determined to be 22 N.

In the final design, the preload was set at 25 N.

The forces that are calculated are now utilized to evaluate whether the equivalent load provides a big enough safety margin compared to the basic static load rating, ensuring the bearing's suitability for this application (**Table 8**).

Similar to the the second distal joint element, the first intermediate joint element shall gravity compensated.

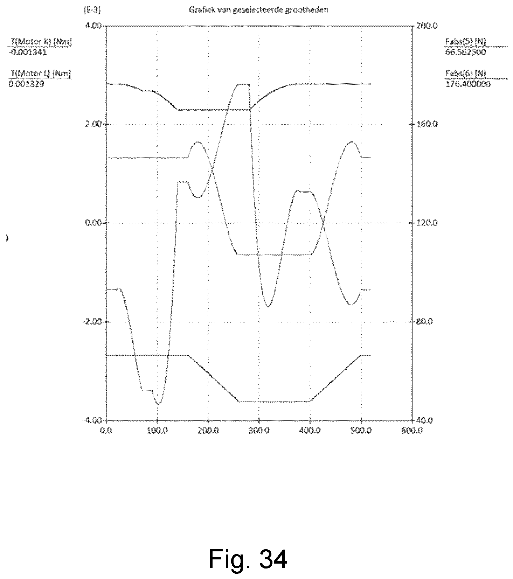

**Fig. 34** shows the results of a SAM simulation focused on the gravity compensator of this joint element.

These result show that the rest torque on the second distal joint element is never more than 2 mNm.

### Bearing calculations for the second intermediate joint element

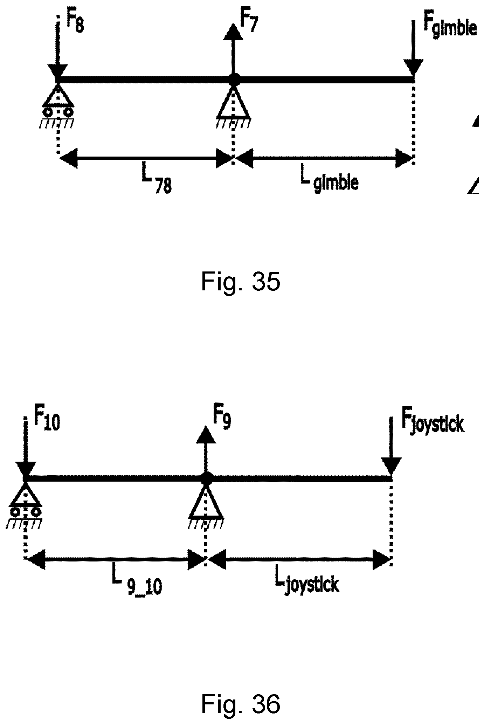

**Fig. 35** shows a free body diagram of forces which have an influence on the axial and radial forces acting on the bearings in the second intermediate joint element.

In the free-body diagram of **Fig. 35****,** the bearings are represented with one modeled as a pin and the other as a roller.

The forces causing an axial and/or a radial load on the joystick are the mass of the gimble.

This mass is 0.39 kg and is located at 60.16mm.

It is assumed that the contact angle for the bearings is 10 deg (*ϕ*). $\left[N\right] = \frac{{F}_{gimble} ⋅ {L}_{gimble}}{{L}_{78}} = \frac{0.39 ⋅ 9.81 ⋅ 60.16}{9.5} = 24.2$ ${F}_{7} = {F}_{8} + {F}_{gimble} = 24.2 + 3.8 = 28.0$ ${F}_{preload} \left[N\right] = {F}_{8} ⋅ tan \left(ϕ\right) = 28.0 ⋅ tan \left(10\right) = 4.9$

The minimum preload of the bearings, based on calculations, is determined to be 4.9 N.

In the final design, the preload was set at 10.2 N. T
he forces that are calculated are now utilized to evaluate whether the equivalent load provides a big enough safety margin compared to the basic static load rating, ensuring the bearing's suitability for this application (**Table 9**).

### Bearing calculations for the first proximal joint element

**Fig. 36** shows a free body diagram of forces which have an influence on the axial and radial forces acting on the bearings in the first proximal joint element.

In the free-body diagram of **Fig. 36****,** the bearings are represented with one modeled as a pin and the other as a roller.

The forces causing an axial and or radial load on the joystick are the mass of the joystick and body of the second distal joint element.

This mass is 0.22 kg and is located at 41.8mm.

It is assumed that the contact angle for the bearings is 10 deg (*ϕ*). The calculations are performed in the most inconvenient position, which means that the joystick is vertical in the world. $\left[N\right] = \frac{{F}_{joystick} ⋅ {L}_{joystick}}{{L}_{9 - 10}} = \frac{0.22 ⋅ 9.81 ⋅ 41.8}{10.5} = 8.6$ ${F}_{9} \left[N\right] = {F}_{10} + {F}_{joystick} = 8.6 ∓ 2.2 = 10.8$ ${F}_{preload} \left[N\right] = {F}_{8} ⋅ tan \left(ϕ\right) = 8.6 ⋅ tan \left(10\right) = 1.5$

The minimum preload of the bearings, based on calculations, is determined to be 1.5 N.

In the final design, the preload was set at 10.2 N.

The forces that are calculated are now utilized to evaluate whether the equivalent load provides a big enough safety margin compared to the basic static load rating, ensuring the bearing's suitability for this application (**Table 10**).

### Bearing calculations for the second proximal joint element

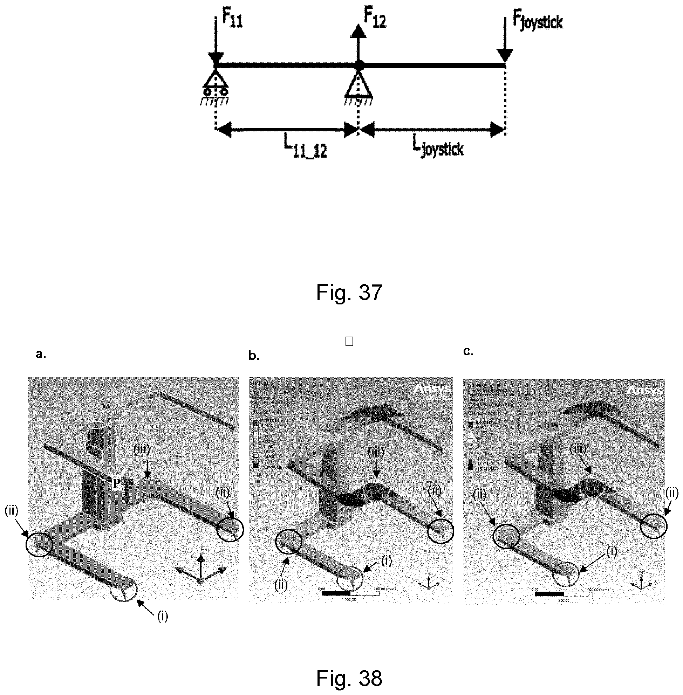

**Fig. 37** shows a free body diagram of forces which have an influence on the axial and radial forces acting on the bearings in the second proximal joint element.

In the free-body diagram of **Fig. 37****,** the bearings are represented with one modeled as a pin and the other as a roller.

The forces causing an axial and or radial load on the joystick are the mass of the joystick.

This mass is 0.07 kg and is located at 71.86mm.

It is assumed that the contact angle for the bearings is 10 deg (*ϕ*). The calculations are performed in the most inconvenient position, which means that the joystick is horizontal in the world. ${F}_{11} \left[N\right] = \frac{{F}_{joystick} ⋅ {L}_{joystick}}{{L}_{9 - 10}} = \frac{0.07 ⋅ 9.81 ⋅ 71.86}{16.5} = 3.0$ ${F}_{12} \left[N\right] = {F}_{11} + {F}_{joystick} = 3.0 ∓ 0.07 ⋅ 9.81 = 3.7$ ${F}_{preload} \left[N\right] = {F}_{11} ⋅ tan \left(ϕ\right) = 3.0 ⋅ tan \left(10\right) = 0.53$

The minimum preload of the bearings, based on calculations, is determined to be 0.53 N.

In the final design, the preload was set at 10.2 N.

The forces that are calculated are now utilized to evaluate whether the equivalent load provides a big enough safety margin compared to the basic static load rating, ensuring the bearing's suitability for this application (**Table 11**).

### Material

Convenient materials for the exterior parts of the master robot arm are AlMgSi 0.5 and AlMgSi 1.

In particular, said materials have been considered due to the following properties:
- Lightweight;
- High stiffness;
- Wide availability;
- Ease of machining;
- Creep resistance;
- Minimizes EMC, and
- Cleanability

### Deformation tests for the cart

Tests have been performed to investigate deformation occurring in the cart.

The results of these tests are illustrated in **Figs. 40a-c**.

The simulation setup involves different boundary conditions for the wheels of the cart.

The wheel denoted with (i) is fixed in all directions (X, Y, Z).

The wheels denoted with (ii) are constrained only in the Z-direction, allowing movement in the X and Y directions.

The wheel denoted with (iii) is completely unconstrained.

A force is applied at point P (see arrow) (**Fig. 40a**) with two different magnitudes, namely 250 N and 1000 N, to determine the deflection and behavior of the system.

When a force of 250 N is applied, the maximum deflection at the point of force is 3.8 mm, and the back wheel denoted with (iii) rises by 2.1 mm (**Fig. 40b**).

Under a force of 1000 N, the deflection at the point of force increases to 15.5 mm, and the raise of the back wheel is 8.5 mm (**Fig. 40c**).

### Based on this simulation it is possible to calculate the stiffness at point P.

The results of the deformation test and the calculated stiffness are shown in **Table 12.**

### Equivalent stress tests for the cart

Tests have been performed to investigate equivalent stress occurring in the cart.

The results of these tests are illustrated in **Figs. 41a-c.**

The material used in the simulation has a yield strength of 235 MPa.

The analysis shows a slight concentration of peak stresses at the corners.

These peaks are likely artifacts of the finite element method (FEM) simulation, potentially caused by mesh size or local discretization effects.

As these stress concentrations do not represent the overall behavior of the material and are numerical artifacts, they will be ignored in the evaluation of the results.

Therefore, this simulation shows that the material that has been chosen for the chassis of the cart can withhold 100 kg at point P without the material having permanent changes.

**Table 1 - Joystick pinching force**

| **Open** | | |
|---|---|---|
| | *Lever 1* | *Lever 2* |
| **phi [deg]** | 41.7 | 41.6 |
| **AB [mm]** | 43.9 | 46.9 |
| **AD [mm]** | 89.8 | 89.8 |
| **Cspring [N/mm]** | 5.5 | 5.5 |
| **Compression [mm]** | 3.5 | 3.5 |
| **Ffinger [N]** | 4.17 | 4.44 |

| **Closed** | | |
|---|---|---|
| | *Lever 1* | *Lever 2* |
| **phi [deg]** | 25 | 25 |
| **AB [mm]** | 43.9 | 46.9 |
| **AD [mm]** | 90.0 | 90.0 |
| **Cspring [N/mm]** | 5.5 | 5.5 |
| **Compression [mm]** | 5.3 | 5.3 |
| **Ffinger [N]** | 3.29 | 3.51 |

**Table 2 - Mechanical limitations for each joint element in a left and right master robot arms**

| | **Left arm range** | **Right arm range** |
|---|---|---|
| First idistal joint element | [0][º] - [47][º] | [-47][º] - [0][º] |
| Second distal joint element | [0][º] - [45.5][º] | [0][º] - [45.5][º] |
| First intermediate joint element | [90][º] - [131][º] | [90][º] - [131][º] |
| Seconf intermediate joint element | [-125.4][º] - [35.4][º] | [-35.4][º] - [125.4][º] |
| First proximal joint element | [-80][º] - [74][º] | [-80][º] - [74][º] |
| Second proximal joint element | [∞][º] | [∞][º] |

**Table 3 - Maximum angular error introduced by parallel misalignment**

| **Encoder type** | **Pos1 angular error [deg]** | **Pos2 angular error [deg]** | **Pos3 angular error [deg]** |
|---|---|---|---|
| **Aura(^{™}) 18** | 3 | 3 | 5 |

**Table 4 - Tip position accuracy for the first three joint elements**

| **Joint element** | **Lenght [mm]** | **Position accuracy [µm]** |
|---|---|---|
| First distal joint element | 648 | 15.532 |
| Second distal joint element | 694 | 16.626 |
| First intermediate joint element | 350 | 8.389 |

**Table 5 - Tip position accuracy for the last three joint elements**

| **Joint element** | **Orientation accuracy [rad]** |
|---|---|
| Second intermediate joint element | 2.4E-05 |
| First distal joint element | 2.4E-05 |
| Second distal joint element | 2.4E-05 |

**Table 6 - Usable bearing for the first distal joint element**

| **Usable bearing** | **True** | | | | |
|---|---|---|---|---|---|
| **Input** | | **Table** | | **Results** | |
| **Fr [kN]** | 0.1147 | X | 1 | **P [kN]** | 0.1147 |
| **Fa [kN]** | 0.0343 | Y | 0 | **C [kN]** | 1.1 |
| | | | | **s [-]** | 9.6 |

**Table 7 - Usable bearing for the second distal joint element**

| **Usable bearing** | **True** | | | | |
|---|---|---|---|---|---|
| **Input** | | **Table** | | **Results** | |
| **Fr [kN]** | 0.2875 | X | 0.56 | **P [kN]** | 0.2804 |
| **Fa [kN]** | 0.06 | Y | 1.99 | **C [kN]** | 2.55 |
| | | | | **s [-]** | 9.09 |

**Table 8 - Usable bearing for the first intermediate joint element**

| **Usable bearing** | **True** | | | | |
|---|---|---|---|---|---|
| **Input** | | **Table** | | **Results** | |
| **Fr [kN]** | 0.1246 | X | 1 | **P [kN]** | 0.1246 |
| **Fa [kN]** | 0.025 | Y | 0 | **C [kN]** | 2.91 |
| | | | | **s [-]** | 23.4 |

**Table 9 - Usable bearing for the second intermediate joint element**

| **Usable bearing** | **True** | | | | |
|---|---|---|---|---|---|
| **Input** | | **Table** | | **Results** | |
| **Fr [kN]** | 0.028 | X | 1 | **P [kN]** | 0.028 |
| **Fa [kN]** | 0.0102 | Y | 0 | **C [kN]** | 0.28 |
| | | | | **s [-]** | 10 |

**Table 10 - Usable bearing for the first proximal joint element**

| **Usable bearing** | **True** | | | | |
|---|---|---|---|---|---|
| **Input** | | **Table** | | **Results** | |
| **Fr [kN]** | 0.0086 | X | 1 | **P [kN]** | 0.0086 |
| **Fa [kN]** | 0.0102 | Y | 0 | **C [kN]** | 0.28 |
| | | | | **s [-]** | 32.5 |

**Table 11 - Usable bearing for the second proximal joint element**

| **Usable bearing** | **True** | | | | |
|---|---|---|---|---|---|
| **Input** | | **Table** | | **Results** | |
| **Fr [kN]** | 0.0037 | X | 1 | **P [kN]** | 0.0037 |
| **Fa [kN]** | 0.0102 | Y | 0 | **C [kN]** | 0.28 |
| | | | | **s [-]** | 75.7 |

**Table 12 - Input and output of the deformation of the cart**

| **Input** | **Min [mm]** | **Stiffness min [N/m]** | **Max [mm]** |
|---|---|---|---|
| 250 N | -3.8 | 6.6 e4 | 2.1 |
| 1000 N | -15.5 | 6.5 e4 | 8.5 |

### Reference list

- 100: Robot system

- 200: Master robot arm
- 202: First, distal arm element
- 204: Second, intermediate arm element
- 206: Third, proximal arm element / joystick
- 208: First, distal joint
- 210: Second, intermediate joint
- 212: Third, proximal joint
- 214: First distal joint element
- 216: Second distal joint element
- 218: First intermediate joint element
- 220: Second intermediate joint element
- 222: First proximal joint element
- 224: Second proximal joint element
- 226: Lever(s) (joystick)
- 228: Linkage mechanism (joystick)
- 230: Spring, compression spring (joystick)
- 232: Cap (joystick)
- 234: Bearing(s) (first distal joint element)
- 236: Bearing(s) (second distal joint element)
- 238: Spring (second distal joint element)
- 240: Gravity compensation means (second distal joint element)
- 242: Upper axle (second distal joint element)
- 244: Bearing(s) (first intermediate joint element)
- 246: Lower axle (first intermediate joint element)
- 248: Pretension stack (first intermediate joint element)
- 250: Stroke limiter (first intermediate joint element)
- 252: insert (second intermediate joint element)
- 254: Bearing(s) (second intermediate joint element)
- 256: Spacing (second intermediate joint element)
- 258: Housing (first proximal joint element)
- 260: Bearing stack (second intermediate joint element)
- 262: Body (first intermediate joint element)
- 264: Encoder (first intermediate joint element)
- 266: Bearing(s) (first proximal joint element)
- 268: Spacer (first proximal joint element)
- 270: Shaft (first proximal joint element)
- 272: Bearing stack (first proximal joint element)
- 274: Clock spring structure
- 276: Flat cable (clock spring structure)
- 278: Mounting block(s) (clock spring structure)
- 280: Mounting bracket (clock spring structure)
- 282: Bearing(s) (second proximal joint element)
- 284: Bearing stack (second proximal joint element)
- 286: Optical encoder PCB (second proximal joint element)
- 288: Body (first proximal joint element)
- 290: Slit (joystick)
- 292: Inner shaft
- 294: Magnet
- 296: Prong
- 298: Outer shaft

- 400: Master control unit

- 500: Slave robot arm

- 600: Robot cart

- 700: Cart (master control unit)
- 702: Wheels (swivel wheels)
- 704: Armrest
- 706: Pedal unit
- 708: Mounting interface
- 710: (First) pedal
- 712: (Second) pedal
- 714: Platform
- 716: Housing for the microscope pedal
- 718: Holding means
- 720: Button
- 722: Height adjustment means
- 724: Mounting means
- 726: Graphical User Interface (GUI)

## Claims

1. A master robot arm (200) for a master control unit (400) of a surgical, microsurgical or super-microsurgical robot system (100), said master robot arm (200) at least comprising:
a first, distal arm element (202);
a second, intermediate arm element (204);
a first distal joint (208) having a first distal joint element (214) configured to perform a first roll movement, and a second distal joint element (216) configured to perform a first pitch movement, and
a second, intermediate joint (210) having a first intermediate joint element (218) configured to perform a second pitch movement, and a second intermediate joint element (220) configured to perform a second roll movement.

2. The master robot arm (200) of claim 1,
**characterized in that**
the master robot arm (200) further comprises:
a third, proximal arm element (206), providing a user interface allowing a user to provide a user input for the master robot arm (200), and
a third, proximal joint (212) having a first proximal joint element (222) configured to perform a third pitch movement, and a second proximal joint element (224) configured to perform a third roll movement,
wherein the distal joint (208), the intermediate joint (210), and the proximal joint (212) provide the master robot arm (200) with six degrees of freedom (DOF).

3. The master robot arm (200) of claim 2,
**characterized in that**
the proximal arm element (206) is in the shape of a joystick (206),
preferably wherein said joystick (206) comprises a pair of hinged levers (226), configured to perform an opening/closing movement with respect to one another, providing the master robot arm (200) with an additional degree of freedom (DOF).

4. The master robot arm (200) of any of the preceding claims,
**characterized in that**
at least one of the joint elements (214, 216, 218, 220, 222, 224) is provided with an encoder,
preferably wherein the encoder is a magnetic encoder or an optical encoder.

5. The master robot arm (200) of claim 5,
**characterized in that**
the first distal joint element (214), and/or the second distal joint element (216), and/or the first intermediate joint element (218) comprises an optical encoder, and/or
the second intermediate joint element (220), and/or the first proximal joint element (222), and/or the second proximal joint element (224) comprises a magnetic encoder.

6. The master robot arm (200) of any of the preceding claims,
**characterized in that**
the distal joint (208) is **characterized by** the following ranges of motion:
first roll movement: +-inf, and/or
first pitch movement: -5° to +30° with respect to the first pitch rotation axis,
and/or
the intermediate joint (210) is **characterized by** the following ranges of motion:
second pitch movement: +62° to +118° with respect to the second pitch rotation axis, and/or
second roll movement: -80° to +80° with respect to the second roll rotation axis,
and/or
the proximal joint (212) is **characterized by** the following ranges of motion:
third pitch movement: -80° to +80° with respect to the third pitch rotation axis, and/or
third roll movement: +-inf.

7. The master robot arm (200) of any of the preceding claims,
**characterized in that**
the master robot arm (200) further comprises at least one gravity compensation means.

8. The master robot arm (200) of claim 7,
**characterized in that**
the at least one gravity compensation means comprises a first gravity compensation means and a second gravity compensation means,
wherein the first gravity compensation means is arranged in the second distal joint element (216), and
wherein the second gravity compensation means is arranged in the first intermediate joint element (218).

9. The master robot arm (200) of any of the preceding claims,
**characterized in that**
the distal arm element (202) has a length between 250 mm and 400 mm, preferably of 350 mm, and/or
the intermediate arm element (204) has a length between 250 mm and 400 mm, preferably of 350 mm.

10. A master control unit (400) for a surgical, microsurgical or super microsurgical robot system (100), at least comprising:
a master robot arm (200) as defined in any of claims 1 to 9, and
a cart (700) allowing for transporting and/or repositioning.

11. The master control unit (400) of claim 10,
**characterized in that**
the cart (700) comprises:
a plurality of wheels (702), preferably wherein said wheels (702) are swivel wheels, more preferably wherein at least two of said wheels (702) are provided with a respective brake, and
a handlebar.
No h

12. The master control unit (400) of claim 11,
**characterized in that**
the cart (700) further comprises a pedal unit (706) comprising at least one pedal (710, 712) arranged on a platform (714),
preferably wherein the pedal unit (706) comprises a first pedal (710) and a second pedal (712), arranged on said platform (714),
preferably wherein the pedal unit (706) further comprises a microscope pedal, arranged on said platform (714).

13. The master control unit (400) of any of claims 10 to 12,
**characterized in that**
the cart (700) further comprises mounting interface (708) for connecting the master robot arm (200) to the cart (700),
preferably wherein the first distal joint element (214) of the master robot arm (200) is arranged within the mounting interface (708),
preferably wherein the cart (700) further comprises a holding means (718) configured for holding the master robot arm (200) in a parking position.

14. The master control unit (400) of any of claims 10 to 13,
**characterized in that**
the master control unit (400) further comprises a second master robot arm (200).

15. A surgical, microsurgical or super-microsurgical robot system (100) at least comprising:
a master control unit (400) as defined in any of claims 10 to 14;
a slave robot arm (500), and
a robot cart (600).
